# EUROPEAN PATENT APPLICATION

(11) **EP 1 839 565 A2**
(43) Date of publication of application: **03.10.2007**
(21) Application number: 07001512.8
(22) Date of filing: 24.01.2007
(51) Int. Cl.: A61B 1/12, A61B 19/00, A61L 2/16, A61L 2/18

(54) **Endoscope washing and disinfecting apparatus and endoscope conduit washing brush cassette**

(30) Priority: 29.03.2006 JP 2006092172
(71) Applicant: OLYMPUS MEDICAL SYSTEMS CORP., Tokyo (JP)
(72) Inventor: Suzuki, Eiri, Shibuya-ku Tokyo 151-0072 (JP); Noguchi, Toshiaki, Shibuya-ku Tokyo 151-0072 (JP); Kobayashi, Kenichi, Shibuya-ku Tokyo 151-0072 (JP); Tomita, Masahiko, Shibuya-ku Tokyo 151-0072 (JP)
(74) Representative: von Hellfeld, Axel

(57) **Abstract**

An endoscope washing and disinfecting apparatus according to the present invention includes an apparatus body having a washing tub for placing and washing an endoscope, a brush forward and reverse mechanism provided with a fluid supply part for supplying a fluid circulating in the apparatus body into a conduit of the endoscope, and a washing brush cassette freely attachable and detachable to/from the brush forward and reverse mechanism, having a washing brush for brush washing the inside of the conduit by moving forward and reversely by the brush forward and reverse mechanism through the fluid supply part. Thus, an inexpensive endoscope conduit washing brush cassette capable of readily changing a washing brush and an easy-to-use endoscope washing and disinfecting apparatus corresponding to the endoscope conduit washing brush cassette can be provided.

## Description

### BACKGROUND OF THE INVENTION

### 1. Field of the Invention

The present invention relates to an endoscope washing and disinfecting apparatus for washing and disinfecting a used endoscope, and an endoscope conduit washing brush cassette used in the washing and disinfecting apparatus.

### 2. Description of the Related Art

An endoscope used for the purpose of examination or treatment of a body cavity is required to be adequately brush washed to clean body fluid, waste material, for example, adhered to not only an outer surface of an insertion part inserted into the body cavity but also adhered in various endoscope conduits such as an air/water supply pipe, a treatment instrument insertion conduit.

In recent years, an endoscope washing and disinfecting apparatus which automatically washes and disinfects an endoscope have been used. A user, before washing and disinfecting an endoscope by the endoscope washing and disinfecting apparatus, has to roughly wash an outer surface of the endoscope and brush and wash various endoscope conduits.

Especially, treatment instrument insertion conduits also function as suction conduits for sucking waste materials in the body cavity, and to the conduits, body fluid, waste material, for example, easily adhere. Accordingly, the conduits are adequately brushed and washed.

Brushing and washing the inside of such various endoscope conduits is very troublesome. Thus, apparatuses and instruments for readily brushing and washing the inside of the endoscope conduits with a washing brush have been proposed.

As an example of them, for example, in Japanese Unexamined Patent Application Publication No. 7-194533, an endoscope washing instrument for moving a washing brush in a forward and backward direction and turning the washing brush is disclosed. Moreover, for example, in Japanese Unexamined Patent Application Publication No. 2004-254806, an endoscope conduit washing apparatus capable of electrically brushing and washing an endoscope is disclosed.

However, the instrument disclosed in Japanese Unexamined Patent Application Publication No. 7-194533 and the apparatus disclosed in Japanese Unexamined Patent Application Publication No. 2004-254806 simply brush and wash various endoscope conduits. For example, with the endoscope washing and disinfecting apparatus described above, if an endoscope is washed and disinfected, the instrument and the apparatus can be used only for rough washing of the endoscope.

Accordingly, the user, even if the endoscope washing and disinfecting apparatus is used, at the time of the rough washing, using the above-described instrument and the above-described apparatus, the various endoscope conduits are brushed and washed, and such work is troublesome.

Moreover, also, in view of good hygiene, single use of the washing brush is preferable. Accordingly, while the user has to install a long washing brush to the above-described instrument and apparatus and change the washing brush, the user has to discard the used contaminated long washing brush with due caution.

Accordingly, in view of the above, the present invention has been made to provide an inexpensive endoscope conduit washing brush cassette capable of readily changing a washing brush and an easy-to-use endoscope washing and disinfecting apparatus corresponding to the endoscope conduit washing brush cassette.

### SUMMARY OF THE INVENTION

An endoscope washing and disinfecting apparatus according to the present invention includes an apparatus body having a washing tub for placing and washing an endoscope, a brush forward and reverse mechanism provided with a fluid supply part for supplying a fluid circulating in the apparatus body into a conduit of the endoscope, and a washing brush cassette freely attachable and detachable to/from the brush forward and reverse mechanism, having a washing brush for brush washing the inside of the conduit by moving forward and reversely by the brush forward and reverse mechanism through the fluid supply part.

Further, an endoscope conduit washing brush cassette includes a washing brush having a flexible shaft provided with a washing brush part for washing a conduit of an endoscope at a tip, and a housing case for housing the washing brush having a connection part freely attachable and detachable to/from an endoscope washing and disinfecting apparatus while functioning as an opening part for feeding out the shaft.

According to the present invention having such a structure, an inexpensive endoscope conduit washing brush cassette capable of readily changing a washing brush and an easy-to-use endoscope washing and disinfecting apparatus corresponding to the endoscope conduit washing brush cassette can be provided.

The above and other objects, features and advantages of the invention will become more clearly understood from the following description referring to the accompanying drawings.

### BRIEF DESCRIPTION OF THE DRAWINGS

Fig. 1 is a view illustrating an external view of an endoscope washing and disinfecting apparatus in a state in which a top cover is opened according to an embodiment of the present invention;
Fig. 2 is a view illustrating an external view of an endoscope washing and disinfecting apparatus in a state in which a top cover is closed according to the embodiment of the present invention;
Fig. 3 is a plan view illustrating an endoscope conduit washing brush cassette according to the embodiment of the present invention;
Fig. 4 is a cross sectional view illustrating an internal part of an endoscope conduit washing brush cassette according to the embodiment of the present invention;
Fig. 5 is a top plan view illustrating an endoscope conduit washing brush forward and reverse mechanism to which an endoscope conduit washing brush cassette is mounted according to the embodiment of the present invention;
Fig. 6 is a view illustrating an endoscope conduit washing brush forward and reverse mechanism to which an endoscope conduit washing brush cassette is mounted viewed from the back direction according to the embodiment of the present invention;
Fig. 7 is a view illustrating an endoscope conduit washing brush forward and reverse mechanism to which an endoscope conduit washing brush cassette is mounted viewed from the right-back direction according to the embodiment of the present invention;
Fig. 8 is a view illustrating a washing brush cassette according to the embodiment of the present invention;
Fig. 9 is a cross sectional view illustrating an internal part of a washing brush cassette according to the embodiment of the present invention;
Fig. 10 is a view for explaining how to mount an endoscope conduit washing brush cassette to an endoscope conduit washing brush forward and reverse mechanism according to the embodiment of the present invention;
Fig. 11 is a view illustrating an endoscope conduit washing brush forward and reverse mechanism to which an endoscope conduit washing brush cassette is mounted viewed from the left-back direction according to the embodiment of the present invention; and
Fig. 12 is a view illustrating a structure of a roller guide in a brush forward and reverse part according to the embodiment of the present invention.

### DETAILED DESCRIPTION OF THE PREFERRED EMBODIMENTS

Hereinafter, with reference to the drawings, an endoscope conduit washing brush cassette and an endoscope washing and disinfecting apparatus corresponding to the endoscope conduit washing brush cassette according to the present embodiment will be described.

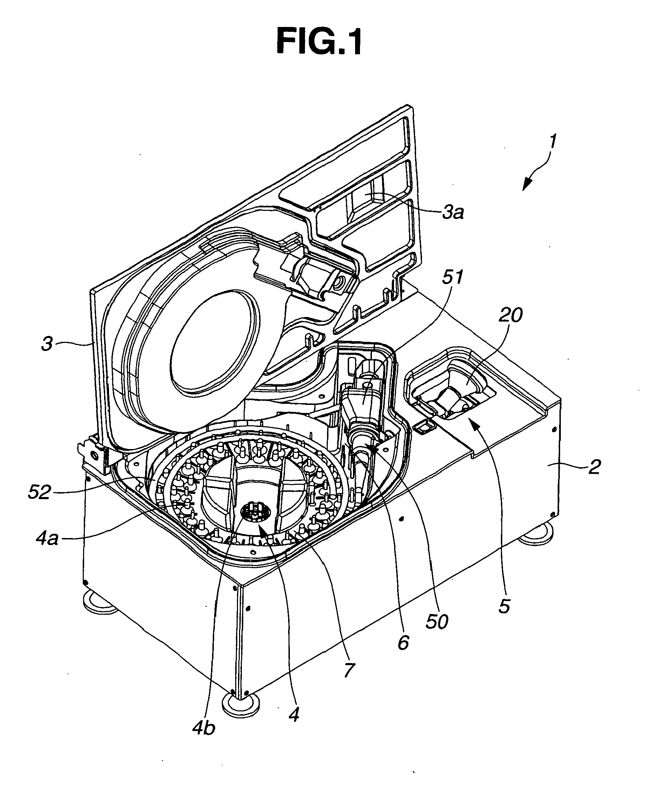

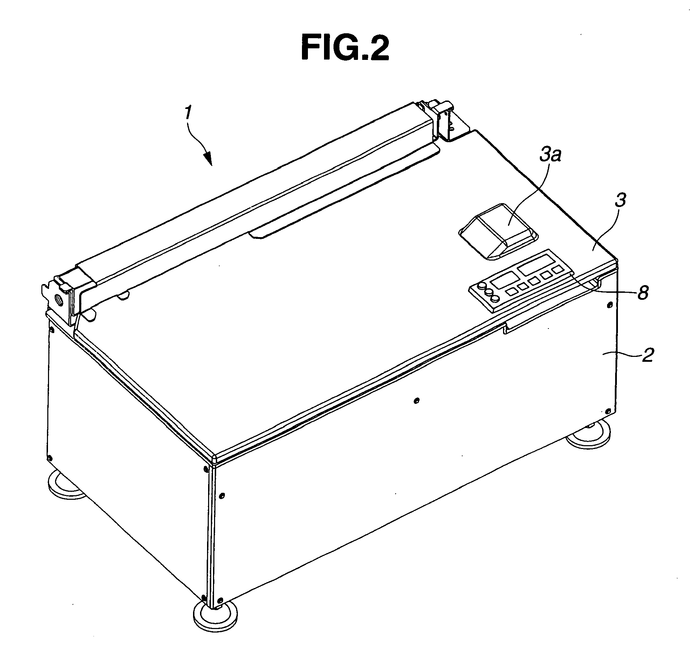

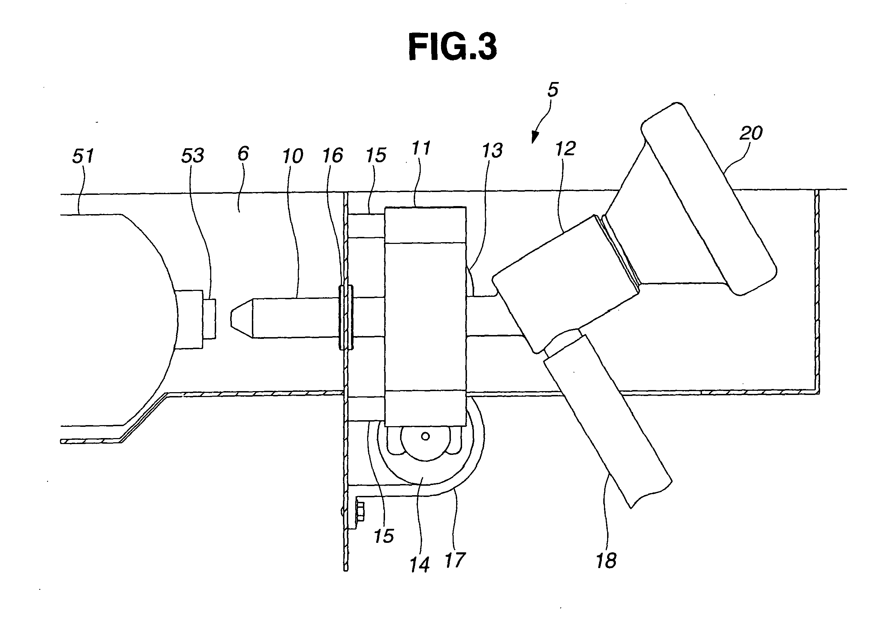

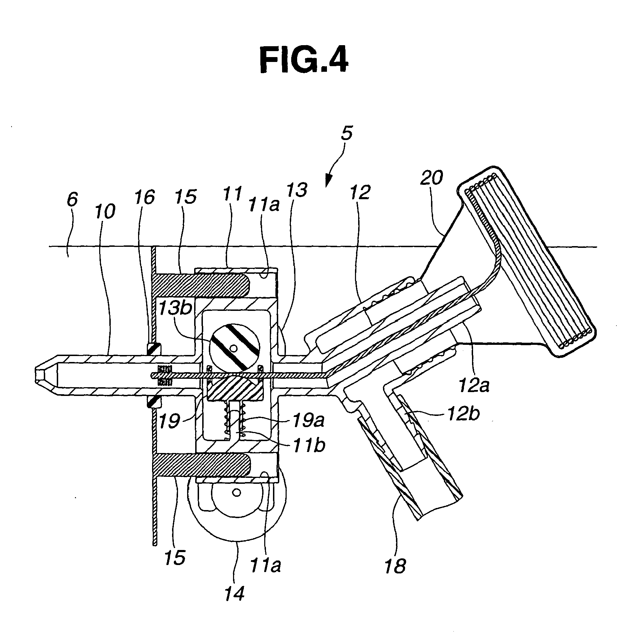

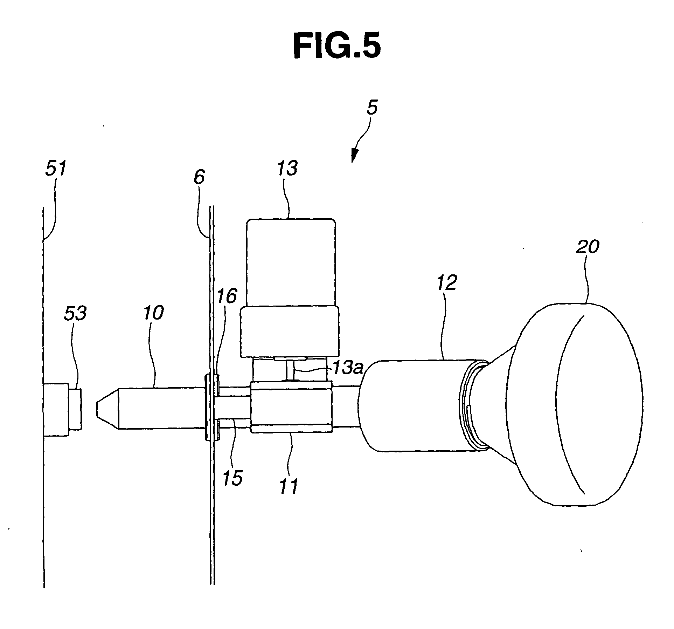

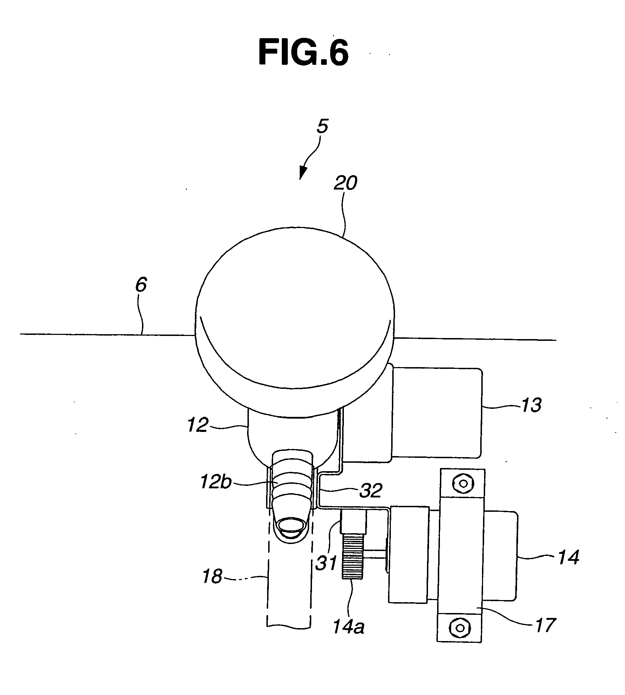

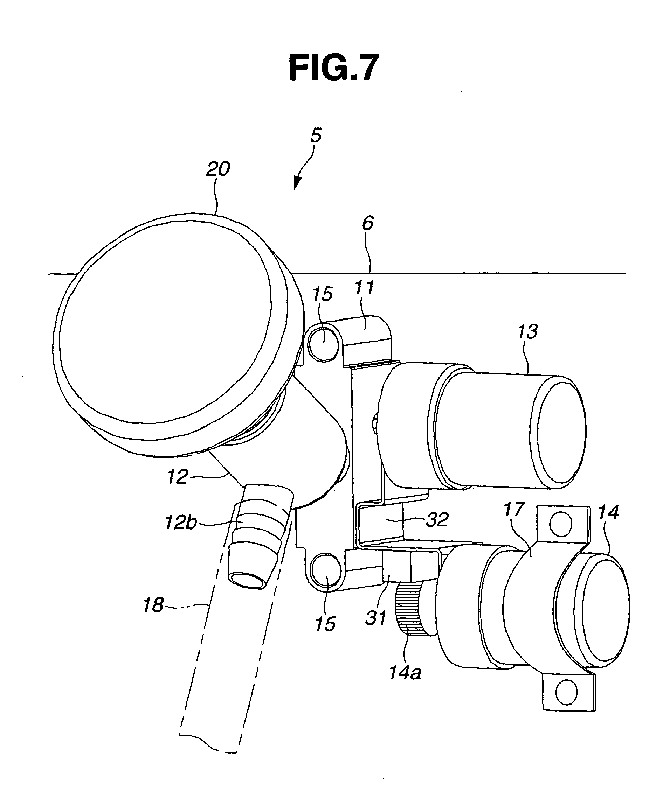

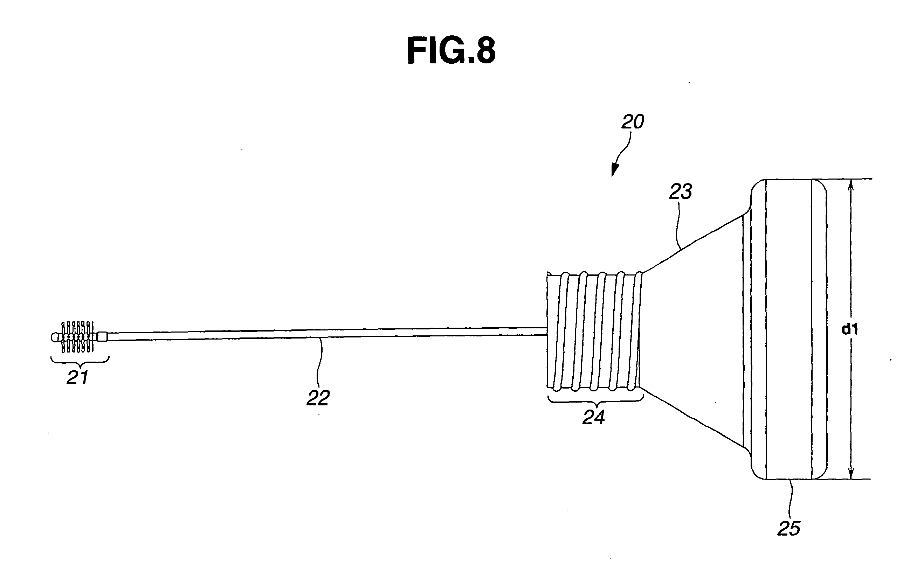

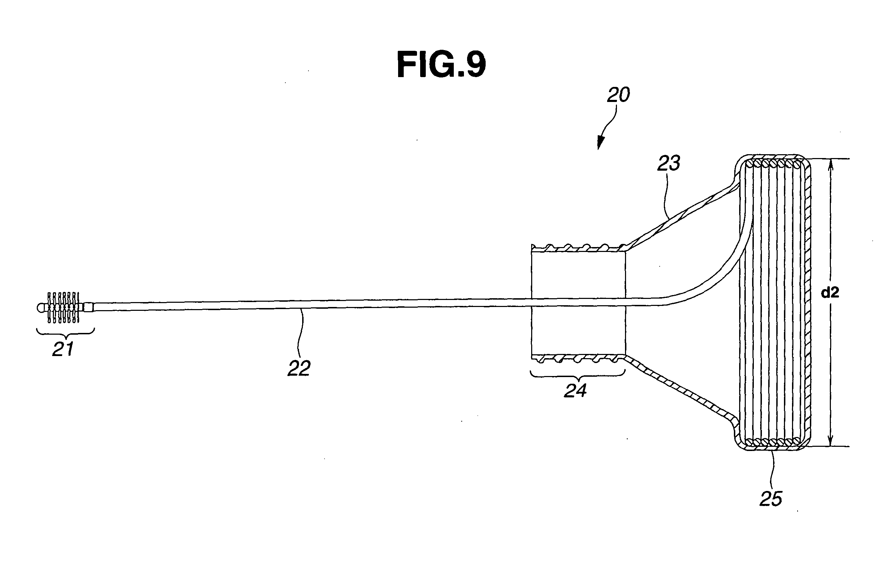

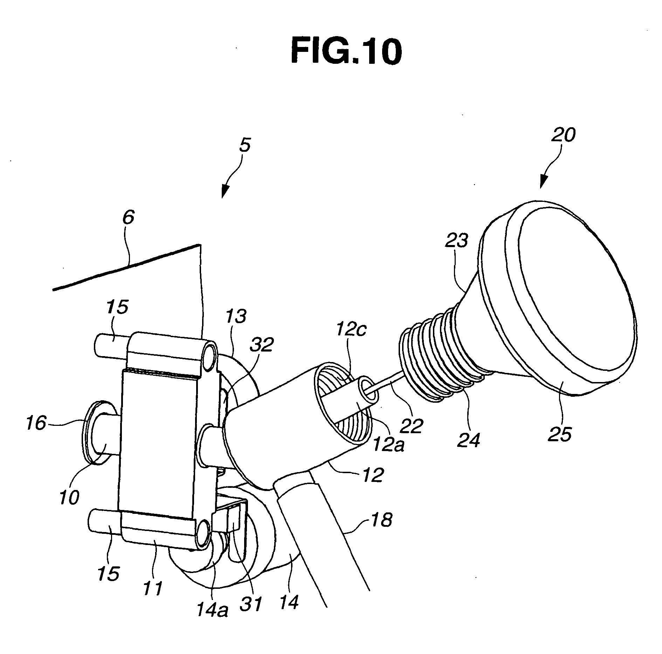

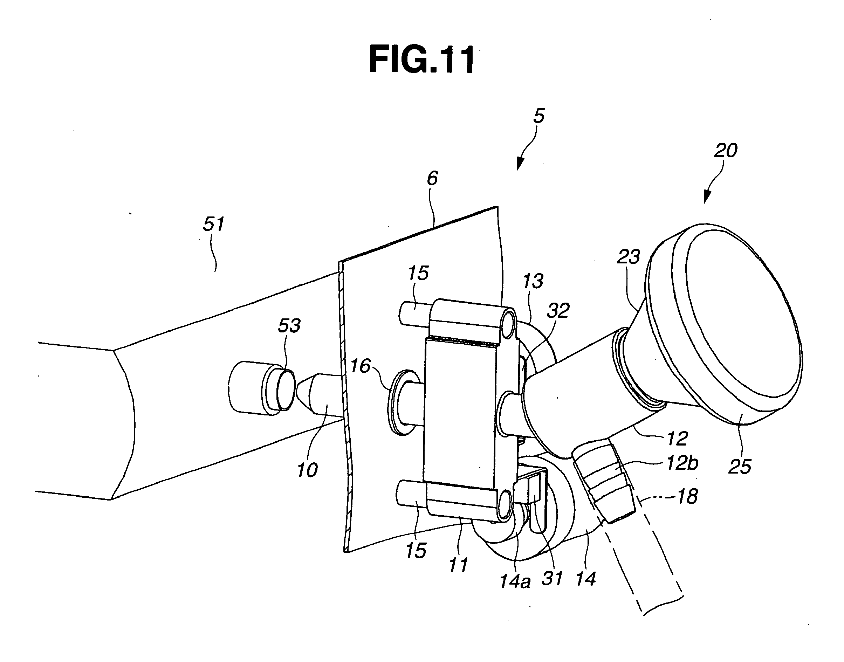

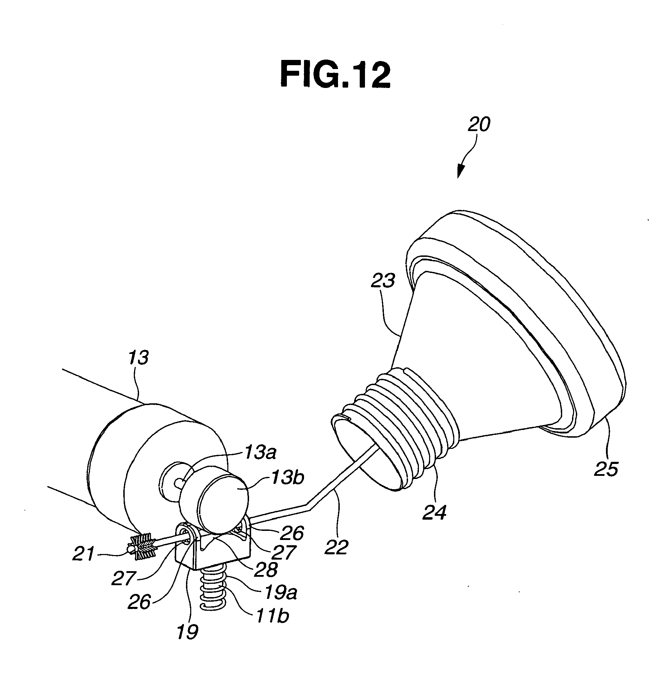

In Figs. 1 to 12 according to the embodiment, Fig. 1 is a view illustrating an external view of an endoscope washing and disinfecting apparatus in a state in which a top cover is opened, Fig. 2 is a view illustrating an external view of an endoscope washing and disinfecting apparatus in a state in which a top cover is closed, Fig. 3 is a plan view illustrating an endoscope conduit washing brush cassette, Fig. 4 is a cross sectional view illustrating an internal part of an endoscope conduit washing brush cassette, Fig. 5 is a top plan view illustrating an endoscope conduit washing brush forward and reverse mechanism to which an endoscope conduit washing brush cassette is mounted according to the embodiment of the present invention, Fig. 6 is a view illustrating an endoscope conduit washing brush forward and reverse mechanism to which an endoscope conduit washing brush cassette is mounted viewed from the back direction according to the embodiment of the present invention, Fig. 7 is a view illustrating an endoscope conduit washing brush forward and reverse mechanism to which an endoscope conduit washing brush cassette is mounted viewed from the right-back direction according to the embodiment of the present invention, Fig. 8 is a view illustrating a washing brush cassette according to the embodiment of the present invention, Fig. 9 is a cross sectional view illustrating an internal part of a washing brush cassette, Fig. 10 is a view for explaining how to mount an endoscope conduit washing brush cassette to an endoscope conduit washing brush forward and reverse mechanism according to the embodiment of the present invention, Fig. 11 is a view illustrating an endoscope conduit washing brush forward and reverse mechanism to which an endoscope conduit washing brush cassette is mounted viewed from the left-back direction, and Fig. 12 is a view illustrating a structure of a roller guide in a brush forward and reverse part.

As illustrated in Fig. 1, an endoscope washing and disinfecting apparatus 1 according to the embodiment includes main components, that is, an apparatus body 2 and top cover 3 which is a lid body freely openable and closable on an upper surface of the apparatus body 2.

In the apparatus body 2, an endoscope 50 is placed on an upper surface part, and a washing and disinfecting tub (hereinafter, simply referred to as a washing tub) 4 for washing and disinfecting and an endoscope conduit washing brush forward and reverse mechanism (also referred to as a washing brush feeding mechanism, and hereinafter, referred to as a brush forward and reverse mechanism) 5 are exposed and provided.

The washing tub 4 includes an operation part placing tub 6 which is formed to fit with a shape of an operation part 51 of the endoscope 50 and an insertion part placing tub 7 for circularly placing an insertion part 52 of the endoscope 50. To the insertion part placing tub 7, a plurality of holding bodies 4a for separating and holding the insertion part 52 in a predetermined distance and in a central part, a washing case installation part 4b for installing a washing case (not shown) for housing buttons, a forceps plug, for example, and washing and disinfecting the buttons, the forceps plug, with the endoscope 50 are provided.

The brush forward and reverse mechanism 5 is arranged in the vicinity of the operation part placing tub 6. The brush forward and reverse mechanism 5 moves forward and reversely against the operation part 51 so that the brush forward and reverse mechanism 5 can be connected or disconnected to/from an endoscope conduit which is arranged in the endoscope 50, in this embodiment, a conduit adaptor 53 of an instrument insertion conduit which also functions as a suction conduit.

To the top cover 3 of the endoscope washing and disinfecting apparatus 1, a cassette cover part 3a is formed so that the brush forward and reverse mechanism 5 does not abut against a back surface part in a closed state. The top cover 3 is formed of a transparent or translucent material and the inside of the washing tub 4 can be observed in a closed state.

Further, as illustrated in Fig. 2, on an upper surface of the top cover 3, an operation instruction part 8 capable of carrying out various operations such as start, stop, display of various steps, elapsed time display, setting of washing and disinfecting step is provided.

In the endoscope washing and disinfecting apparatus 1, in the apparatus body 2, a conduit structure in which an electromagnetic valve, a check valve, and the like are disposed, a pump, and a compressor are embedded to circulate washing fluid, antiseptic solution, rinsing water, alcohol, and air for washing and disinfecting the endoscope 50 into the washing tub 4, and the brush forward and reverse mechanism 5 at various steps (washing, disinfection, alcohol flash, and dewatering). Further, in the apparatus body 2, a control part for driving and stopping each of the above-described electric equipments according to a program of each of the above-described various steps is embedded.

Each fluid of the washing fluid, antiseptic solution, and alcohol is stored in each tank provided in the apparatus body 2. To the endoscope washing and disinfecting apparatus 1, tap water used for rinsing, and diluting the washing fluid and antiseptic solution is supplied from a water tap with a hose (not shown), for example, connected to the apparatus body 2.

Now, with reference to Figs. 3 to 6, the brush forward and reverse mechanism 5 will be described.

As illustrated in Figs. 3 to 5, the brush forward and reverse mechanism 5 according to the embodiment includes a nozzle 10 which is a tubular fluid supply part formed of a hard metal or a synthetic resin, or the like, a brush forward and reverse part 11 which is provided in a middle part of the nozzle 10, a substantially cylindrical-shaped brush cassette application part 12 which is consecutively provided at a base end of the nozzle 10 formed of a hard metal or a synthetic resin, or the like, and its tip side is blocked, a brush forward and reverse motor 13 in which a motor axis 13a is inserted through the brush forward and reverse part 11, and a nozzle feeding motor 14 for moving the nozzle 10 forward and reversely with respect to the operation part 51 of the endoscope 50.

The nozzle 10 has a substantially cone shape at the tip part and the tip part extends into the operation part placing tub 6. The nozzle 10 is inserted into a seal 16 provided to a wall part of the operation part placing tub 6, and by the seal 16, even if the nozzle 10 moves forward and reversely to the operation part 51, the washing tub 4 including the operation part placing tub 6 is kept in air tight (water tight).

The brush forward and reverse part 11, as illustrated in Fig. 4, is a hollow box body formed of a metal or a synthetic resin. The brush forward and reverse part 11 has two guide openings 11a which are inserted through in lateral direction of top and bottom respectively, and a substantially bar-shaped protruded part 11b which protrudes from below into an internal space formed at substantially center.

In the internal space, a roller 13b formed of an elastic member which is fixed to the motor axis 13a of a brush forward and reverse motor 13, and a roller guide 19 which is freely movably externally inserted to the protruded part 11b are provided. The roller guide 19 is always biased to a roller 13b side by a pressure spring 19a.

Into the two guide openings 11a of the brush forward and reverse part 11, guide pins 15 which are extended in a lateral direction from an outer wall of the operation part placing tub 6 are inserted respectively. Thus, the brush forward and reverse mechanism 5 is guided straight by the two guide pins 15.

The brush cassette application part 12 has a tubular brush insertion part 12a which is integrally formed along a long axis at substantially center, and a substantially tubular tube connection part 12b which is provided in a protruding state from a side peripheral part, and integrally formed.

As shown in the drawing, in the brush cassette application part 12, the passageway of the tubular brush insertion part 12a is not internally communicated with a space formed at an outer peripheral side, and has a length that the tubular brush insertion part 12a protrudes from an opening part. The space of the outer peripheral side communicates with the tube connection part 12b.

To the tube connection part 12b, a fluid supply tube 18 is connected. The fluid supply tube 18 is connected to a fluid supply tube (not shown) in an internal part of the apparatus body 2, the washing fluid, the antiseptic solution, the alcohol, or the air which circulates in each conduit net in the apparatus body 2 at each predetermined step is supplied into the brush cassette application part 12.

As illustrated in Figs. 6 and 7, the brush forward and reverse part 11 and the brush forward and reverse motor 13 are integrally fixed by a plate-shaped fixing metallic material 32 which is formed in a substantially hat shape. To a lower surface of the fixing metallic material 32, a rack gear 31 which engages a pinion gear 14a of the nozzle feeding motor 14 is provided.

Thus, with a drive of the nozzle feeding motor 14, the tip of the nozzle 10 of the brush forward and reverse mechanism 5 moves forward and reversely toward the operation part 51 of the endoscope 50. The nozzle feeding motor 14 is fixed to the outer wall of the operation part placing tub 6 of the washing tub 4 by a fixing member 17.

The nozzle feeding motor 14 according to the embodiment can be embedded with a decelerator so that a predetermined forward and reverse speed of the nozzle 10 can be obtained, or, without being embedded with the decelerator, can be structured to engage the rack gear 31 with a combination of deceleration gear rows.

In the brush forward and reverse mechanism 5, the internal spaces of the nozzle 10 and the brush forward and reverse part 11 and the brush insertion part 12a of the brush cassette application part 12 are communicate with each other. To a base end opening part of the brush cassette application part 12, a freely attachable and detachable brush cassette 20 which will be described below is connected by screwing.

Next, with reference to Figs. 8 and 9, the washing brush cassette 20 of the embodiment will be described.

As illustrated in Figs. 8 and 9, the washing brush cassette 20 includes a washing brush part 21, a shaft wire 22 to which the washing brush part 21 is provided at a tip part, formed of a metal, or polyethylene such as PP, PE, and has flexibility, and a cassette body 23 which is a washing brush housing case formed of a material which has resistance to the washing fluid and the antiseptic solution such as plastic, synthetic resin of transparent or translucent. In this embodiment, the washing brush part 21 and the shaft wire 22 constitute a washing brush.

The shaft wire 22 is longer in its length than an endoscope conduit such as a treatment instrument insertion passageway provided from the operation part 51 to the insertion part 52 of the endoscope 50. The shaft wire 22 is wound and housed in the cassette body 23 from the base end.

The cassette body 23 has a opened substantially cylindrical shape at a tip, and is a hollow case body which has a connection part 24 which has a male screw part formed on an outer peripheral part, and a substantially cylindrical-shaped shaft housing part 25 which is integrally formed consecutively from the connection part 24 and expanded in a cone shape, and the shaft wire 22 is wound and housed.

In the cassette body 23, preferably, an outside diameter d1 (see Fig. 8) of the shaft housing part 25 is, for example, 12 cm or less (d1 ≤ 12 cm), and an inside diameter d2 (see Fig. 9) is, for example, 4 cm or more (d2 ≥ 4 cm).

The brush cassette of the embodiment structured as described above has a simple structure, and accordingly, can be manufactured at a very low price. The washing brush cassette 20 of the embodiment is packed in a sterilized package so as not to come in contact with outside air in consideration of the aspect of good hygiene before use, and it is preferable to be used as a disposable (single use product). Of course, the washing brush cassette 20 can be a type usable for several times.

As illustrated in fig. 12, the roller guide 19 of the embodiment provided in the brush forward and reverse part 11 has two shaft guides 26 which protrude from upper part front and back ends to be a roller 13b side respectively. At the centers of these shaft guides 26, hole parts 27 are formed.

Accordingly, by inserting through the hole parts 27 of the two shaft guides 26, the shaft wire 22 is inserted through and held at two points, and ensured to be pressed in the vicinity of substantially center of a roller surface of the roller 13b. Thus, for example, due to the wound housing in the cassette body 23, even if the shaft guide 26 is skewed, the shaft guide 26 is ensured to move forward and reversely in conjunction with the turn of the roller 13b. As a result, the washing brush part 21 can also be ensured to move forward and reversely in the endoscope conduit.

The roller guide 19 has a curbed surface 28 of which a surface opposed to the roller 13b protrudes. Accordingly, friction between the shaft wire 22 which slides to the roller guide 19 is reduced.

In thus structured washing brush cassette 20 of the embodiment, first, by a user, as illustrated in Fig. 10, the shaft wire 22 is inserted into the brush cassette application part 12 from the washing brush part 21. Then, the user, as illustrated in Fig. 4, inserts the washing brush part 21 into the nozzle 10 until the shaft wire 22 passes through between the roller 13b and the roller guide 19 in the brush forward and reverse part 11.

Then, as illustrated in Fig. 11, the user screws the connection part 24 to a female screw part 12c (see Fig. 11) formed on the inner peripheral surface of the brush cassette application part 12. Thus, the washing brush cassette 20 is mounted on the brush forward and reverse mechanism 5.

In the cassette body 23, as described above, since the outside diameter d1 of the shaft housing part 25 is 12 cm or less, a general adult can easily hold the shaft housing part 25, and further, can easily carry out the turning operation when screwing the connection part 24 on/off the brush cassette application part 12.

As described above, the user, while mounting the washing brush cassette 20 on the brush forward and reverse mechanism 5, as illustrated in Fig. 1, after placing the endoscope 50 in the washing tub 4, as illustrated in Fig. 2, closes the top cover 3 on the apparatus body 2, and turns on a start button on the operation instruction part 8.

Then, the endoscope washing and disinfecting apparatus 1, according to a predetermined program, washes and disinfects the outer surface of the endoscope 50 and the endoscope conduit, in this case, brushes and washes the instrument insertion conduit by forward and reverse movement of the washing brush part 21.

In the brush washing, by the control part in the apparatus body 2, the brush forward and reverse motor 13 and the nozzle feeding motor 14 are driven and controlled. Specifically, first, the nozzle feeding motor 14 is driven, and the nozzle 10 moves forward toward the conduit adaptor 53 provided to the operation part 51 of the endoscope 50 and connected. Then, the brush forward and reverse motor 13 is repeatedly turn driven, and by feeding in and out the shaft wire 22 pressed between the roller 13b and the roller guide 19, the washing brush part 21 moves forward and reversely in the instrument insertion conduit.

Then, the washing fluid and rinsing water circulating in the apparatus body 2 flows into the washing brush cassette 20 from the fluid supply tube 18 through the brush cassette application part 12. The washing fluid and the rinsing water flows into the nozzle 10 from the brush insertion part 12a of the brush cassette application part 12 through the brush forward and reverse part 11, and flows into the instrument insertion conduit.

In the cassette body 23, as described above, since the inside diameter d2 of the shaft housing part 25 is 4 cm or more, the shaft wire 22 is prevented from being folded extremely or being skewed extremely. When the shaft wire 22 is reversely moved, the shaft wire 22 is housed in the cassette body 23 from the base end side. Accordingly, if the inside diameter d2 of the shaft housing part 25 is too small, the shaft wire 22 cannot be smoothly wound and housed.

As described above, the endoscope washing and disinfecting apparatus 1 of the embodiment brushes and washes the instrument insertion conduit of the endoscope 50. Then, while the user can visually check the washing disinfecting state of the endoscope 50 because the top cover 3 of the endoscope washing and disinfecting apparatus 1 is transparent or translucent, since the cassette body 23 of the washing brush cassette 20 is also transparent or translucent, the user can also check a state of the forward and reverse movement of the shaft wire 22 to which the washing brush part 21 is connected.

When the brush washing by the washing brush part 21 is finished, the brush forward and reverse motor 13 is drive-controlled so that the shaft wire 22 is housed in the cassette body 23. The brush forward and reverse motor 13 is driven until a predetermined time for the washing brush part 21 and the shaft wire 22 to adequately pass through between the roller 13b and the roller guide 19 has passed.

Similarly to the flow of the washing fluid and rinsing water in the washing step and rinsing step, in a state in which the forward and reverse drive of the washing brush part 21 is stopped, the antiseptic solution, the alcohol, or the air flows into the brush forward and reverse mechanism 5 and the washing brush cassette 20 from the fluid supply tube 18 and flows into the instrument insertion conduit of the endoscope 50.

Further, since the antiseptic solution and the rinsing water also flow into the washing brush cassette 20, with the internal part, the washing brush part 21 and the shaft wire 22 are also disinfected and rinsed. Thus, when the washing brush cassette 20 is detached from the brush forward and reverse mechanism 5, the user is prevented from exposure due to the antiseptic solution or waste material.

As a result of the above, the endoscope washing and disinfecting apparatus 1 of the embodiment can automatically brush and wash the endoscope conduit of the used endoscope 50, in the above case, the instrument insertion conduit. Accordingly, the user is free from the troublesome operation, that is, the brush washing of the endoscope conduit carried out at the time of the rough washing of the endoscope 50.

Further, the washing brush cassette 20 of the embodiment, with the structure readily attachable and detachable to the endoscope washing and disinfecting apparatus 1, can save the labor for installing and changing the long washing brush.

Further, the used washing brush cassette 20 is in the state in which the long shaft wire 22 is housed in the cassette body 23, the washing brush cassette 20 can be readily discarded.

As described above, in the present invention, the inexpensive endoscope conduit washing brush cassette capable of readily changing the washing brush and the easy-to-use endoscope washing and disinfecting apparatus corresponding to the endoscope conduit washing brush cassette can be realized.

In the above description, the instrument insertion conduit of the endoscope 50 especially contaminated has been described as an example. However, it is to be understood that the present invention is not limited to the above, various endoscope conduits can be brushed and washed.

The present invention is not limited to the above embodiment, but various modifications can be made without departing from the subject matter of the invention in the stages of reduction to practice. Further, the above embodiment contains inventions in various stages. Various inventions can be extracted from appropriate combinations of a plurality of components to be disclosed.

For example, even though some of all the components shown in the embodiment are deleted, the structure of the remaining components can be extracted as an invention if the problem of the invention can be resolved and the advantage of the invention is obtained.

Having described the preferred embodiments of the invention referring to the accompanying drawings, it should be understood that the present invention is not limited to those precise embodiments and various changes and modifications thereof could be made by one skilled in the art without departing from the spirit or scope of the invention as defined in the appended claims.

## Claims

1. An endoscope washing and disinfecting apparatus comprising:
an apparatus body having a washing tub for placing and washing an endoscope;
a brush forward and reverse mechanism provided with a fluid supply part for supplying a fluid circulating in the apparatus body into a conduit of the endoscope; and
a washing brush cassette freely attachable and detachable to/from the brush forward and reverse mechanism, having a washing brush for brush washing the inside of the conduit by moving forward and reversely by the brush forward and reverse mechanism through the fluid supply part.

2. The endoscope washing and disinfecting apparatus according to Claim 1, wherein the washing brush cassette has a substantially cylindrical-shaped housing part for winding and housing a shaft of the washing brush.

3. The endoscope washing and disinfecting apparatus according to Claim 2, wherein the housing part has an outside diameter set to be 12 cm or less.

4. The endoscope washing and disinfecting apparatus according to Claim 2 or Claim 3, wherein the housing part has an inside diameter set to be 4 cm or more.

5. The endoscope washing and disinfecting apparatus according to any one of Claims 1 to 4, wherein the washing brush cassette is formed of a member of transparent or translucent.

6. An endoscope conduit washing brush cassette comprising:
a washing brush having a flexible shaft provided with a washing brush part for washing a conduit of an endoscope at a tip; and
a housing case for housing the washing brush having a connection part freely attachable and detachable to/from an endoscope washing and disinfecting apparatus while functioning as an opening part for feeding out the shaft.

7. The endoscope conduit washing brush cassette according to Claim 6, wherein the housing case has a substantially cylindrical-shaped housing part for winding and housing the shaft.

8. The endoscope conduit washing brush cassette according to Claim 7, wherein the housing part has an outside diameter set to be 12 cm or less.

9. The endoscope conduit washing brush cassette according to Claim 7 or Claim 8, wherein the housing part has an inside diameter set to be 4 cm or more.

10. The endoscope conduit washing brush cassette according to any one of Claims 6 to 9, wherein the housing case is formed of a member of transparent or translucent.
